(19)

**Europäisches Patentamt**
**European Patent Office**
**Office européen des brevets**

(11) **EP 4 559 379 A1**

(12) # EUROPEAN PATENT APPLICATION

(43) Date of publication:
**28.05.2025 Bulletin 2025/22**

(21) Application number: **23211258.1**

(22) Date of filing: **21.11.2023**

(51) International Patent Classification (IPC):
**A61B 5/00** *(2006.01)*　　**A45D 26/00** *(2006.01)*
**A61B 18/20** *(2006.01)*　　**A61C 17/16** *(2006.01)*
**A61M 1/06** *(2006.01)*

(52) Cooperative Patent Classification (CPC):
**A61B 5/444; A61B 5/0088; A61B 5/0091;**
**A61B 5/441; A61B 5/445; A61B 18/203;**
**A61M 1/064; A61M 1/0697;** A45D 26/00;
A45D 2026/008; A61B 5/0059; A61B 5/489;
A61B 2560/0431; A61C 17/16; A61M 2205/3306;

(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
 • **PAULUSSEN, Elvira Johanna Maria**
  **Eindhoven (NL)**
 • **VAN DER GRAAFF, Leon**
  **Eindhoven (NL)**

 • **DAMODARAN, Mathivanan**
  **Eindhoven (NL)**
 • **JOHNSON, Mark Thomas**
  **Eindhoven (NL)**
 • **GERHARDT, Lutz Christian**
  **Eindhoven (NL)**
 • **VAN LIESHOUT, Ron Martinus Laurentius**
  **Eindhoven (NL)**
 • **PERRONE, Antonio Luigi**
  **Eindhoven (NL)**

(74) Representative: **Philips Intellectual Property &**
**Standards**
**High Tech Campus 52**
**5656 AG Eindhoven (NL)**

(54) **PERSONAL CARE OR PERSONAL HEALTH DEVICE**

(57)　　A device is for application against a body of a subject, for implementing a primary personal care or personal health function. An imaging system is integrated into the device for imaging beneath the surface of a part of the body of the subject. The imaging system implements light sheet microscopy to generate a detected image, from which screening of health conditions or early detection of abnormalities or disease onset can be made, in particular relating to the vasculature beneath the surface of the part of the body.

FIG. 2

EP 4 559 379 A1

**(Cont. next page)**

(52) Cooperative Patent Classification (CPC): (Cont.)
A61M 2205/587; A61M 2230/00

**Description**

FIELD OF THE INVENTION

This invention relates to personal care or personal heath devices, and in particular relates to providing imaging functionality with such devices, so that they can be used to assess health conditions in addition to providing a separate primary function.

BACKGROUND OF THE INVENTION

It is becoming increasingly common to incorporate smart additional functions into a personal health or personal care device. Examples of a personal care device include an epilator, a shaver, a breast pump, a massager, etc. Examples of personal health device include a light treatment device for skin rejuvenation or a light treatment device for the teeth and gums.

This invention relates in particular to hand-held devices which may be applied to the body (e.g., to the skin, or gums, or teeth).

It is known to incorporate imaging into such devices, for example to image the gums or teeth to assess gum and tooth health, or to use image analysis to inspect the closeness of shaving.

There are however other conditions which only manifest themselves beneath the skin surface.

Superficial vein thrombosis is a blood clot that occurs in veins under the skin (superficial veins). The condition typically happens in the arms or legs and causes inflammation, pain, redness and swelling. This is therefore typically visible at the skin surface.

In deep vein thrombosis (DVT), blood clots develop in the body's deep veins, in particular those that are not visible through the skin. Blood clots in the deep veins are dangerous because they can travel to the lungs and stop blood flow (causing a pulmonary embolism).

Deep vein thrombosis is usually examined and diagnosed with vascular ultrasound which has the disadvantage that an additional application of ultrasound coupling gel is needed for imaging (which add extra hassle to the user). Advice to heal deep vein thrombosis can be applying a warm compress, elevating the limb when resting, taking nonsteroidal anti-inflammatory drugs or wearing compression stockings (if the blood clot is in the leg).

Vein thrombophlebitis can be a sign of a systemic disease like an underlying cardiovascular disease or hormone disruption, so in case of multiple blood clots a more appropriate advice could be undergoing a health check.

Another example of vascular condition is superficial dilation of the tongue vessels, called sublingual varices, or varicose (leg) veins which are twisted, enlarged veins. This can also be related to health status. Smoking, hypertension, atrial fibrillation, ischemic heart disease, myocardial infarction, stroke, other cardiovascular diseases, and hemorrhagic telangiectasia (small, dilated blood vessels which manifest as spider veins) can be an indication of sublingual varices in the tongue.

Due to its numerous health risks, screening, diagnosis and early detection of vein thrombosis is desired when it goes unnoticed by the patient for a long period of time. In an early stage of diagnosis, minor healing methods as described above can be used before vein thrombosis starts to be a serious health concern. When vein thrombosis is caused by other (systemic) health issues, these issues may remain unnoticed if there is not an early diagnosis. The same applies more generally to other conditions which are manifested beneath the skin surface, such as other vascular conditions.

It would be of interest if a system for analysis of preferably vascular conditions beneath the skin surface could be performed, and using an existing device of the user so that there is little additional burden for the user, and without the need for ultrasound imaging.

SUMMARY OF THE INVENTION

The invention is defined by the claims.

According to examples in accordance with an aspect of the invention, there is provided a handheld personal care or personal health device, comprising:

a user interfacing element for application against a body of a subject, for implementing a primary personal care or personal health function; and
an imaging system for imaging beneath the surface of a part of the body of the subject, wherein the imaging system comprises:

an illumination source for generating a light sheet and directing the light sheet towards the part of the body such that a waist of the light sheet is at a region of interest beneath the surface of the part of the body; and
a detector for detecting light from the waist of the light sheet from a direction substantially perpendicular to a plane of the light sheet and generating a detected image.

This device is used to perform a main personal health or care function, but additionally has an integrated imaging system for imaging beneath the tissue surface. By imaging beneath the tissue surface, issues relating to the vasculature of the subject using the handheld device may be assessed. This enables a subject to have vasculature conditions assessed while they perform their personal care routine.

The imaging system implements the principle of light sheet microscopy. It for example uses light in the infrared spectrum. More generally, a wavelength of light in the visible/near infrared range from 300 nm to 3000 nm may be used. The lower visible light wavelengths (e.g., 380nm blue) can be also used for fluorescent light sheet microscopy for excitation.

This imaging setup has multiple benefits. A single 2D plane of the sample can be imaged quickly, meaning fast dynamic processes can be seen. By exciting only one plane at the waist of the illumination beam and at a desired position of the sample, there is less out-of-focus light which improves the signal to noise ratio. Furthermore, by imaging only at a well-defined depth defined by the dimension and plane of the light sheet, the image data volume can be reduced.

The illumination source for example comprises a light source and illumination optics (suitable lenses, optical filters etc.) for generating the light sheet and the detector comprises detection optics focused at the waist of the light sheet and a detector. This provides a light sheet microscopy configuration. The detection optics for example comprises an objective lens focused at the waist of the light sheet.

One or both of the illumination optics and the detection optics may comprise optical filters. Optical filters may for example be used to implement fluorescence light sheet imaging. Filters may also be used to transmit certain spectral ranges for improving image quality. For example, a cut-off filter in the range of 600 nm 700 nm may be used to image fungal tissue.

In one implementation, an additional sensor (e.g., a vibration sensor, accelerometer, gyroscope) is provided for sensing an incline of the part of the body of the subject and a controller may then be used for adjusting the illumination optics and/or the detection optics in dependence on the sensed incline. In this way, if the surface being imaged is inclined compared to the desired orientation for the optical system, the optical system may be reconfigured to maintain an in-focus image. Filter settings may also be adjusted.

In another implementation, a sensor is again provided for sensing an incline of the part of the body of the subject and controls the storage and/or analysis of the detected images in dependence on the sensed incline or else combines image portions to create an overall image based on the sensed incline of the image portions. In this way, image reconstruction is improved by selecting or using only images which have been generated with a configuration giving in-focus images. Furthermore, image acquisition or image storage data volume can be reduced.

In another implementation, a sensor is again provided for sensing an incline of the part of the body of the subject, and a scanning mirror is provided between the detection optics and the detector. A controller then controls the scanning mirror orientation in dependence on an incline of the part of the body of the subject. Thus, the optical path is adapted to take account of an incline of the body surface.

In all cases, the imaging system is for example for imaging the vasculature beneath the surface of a subject.

In a first example, a processor is provided for analyzing the detected image to provide an assessment of deep vein thrombosis. Detection of deep vein thrombosis at an early stage by imaging beneath the skin surface can prevent medical complications.

The device is for example an intense pulsed light module of an epilator, and the primary personal care or personal health function comprises hair removal. Since this type of device is typically used on the legs, it can provide detection of deep vein thrombosis which commonly occurs in the legs.

The intense pulsed light (IPL) epilator device for example has a light delivery window for delivering light flashes, and the illumination source delivers the light sheet through the light delivery window. This provides a compact arrangement, with the light sheet light source integrated into the structure of the epilator.

In a second example, a processor is provided for analyzing the detected image to provide an assessment of sublingual varices. The device may then comprise an oral appliance such as a mouthpiece, night guard, aligner etc.), and the primary personal care or personal health function comprises gum and/or tooth treatment or gum and/or tooth protection.

In a third example, a processor is provided for analyzing the detected image to provide an assessment of early indication of breast vasospasm or inflammation by enlarged breast veins. The device may then comprise a breast pump, and the primary personal care or personal health function comprises breast pump milk extraction.

It will thus be seen that sub-surface image analysis may be used in a variety of personal health or personal care situations to provide detection and/or assessment of conditions associated with the same general body area with which the device interacts during its primary use (such as the legs, mouth, breast).

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows the principle of light sheet microscopy;
Fig. 2 shows the waist length L and width W of the light sheet;
Fig. 3 shows an IPL device including an imaging system for imaging beneath the surface of a part of the body of the subject;
Fig. 4 shows how the imaging system may be implemented in the intense pulsed light module/unit of the IPL device;
Fig. 5 shows an alternative arrangement of the imaging system in the intense pulsed light module/unit of the IPL device;

Fig. 6 shows another alternative arrangement of the imaging system in the intense pulsed light module/unit of the IPL device;

Fig. 7 shows a variation to Fig. 6;

Fig. 8 shows a light sheet imaging configuration for monitoring of sublingual varices using an oral appliance such as aligner or mouthpiece;

Fig. 9 shows a light sheet imaging configuration integrated in a breast shield which may be part of a breast pump for monitoring vasospasm precursors of veins of the areola or breast;

Fig. 10 shows an example of a tilted arrangement;

Fig. 11 shows the use of a compensating scanning mirror to enable different tilts to be tolerated;

Fig. 12 shows an inertial measurement unit (IMU) providing motion sensing information to a controller to control a tilt stage; and

Fig. 13 shows a method to store and/or analyze images only if the hand-held device is at a desired orientation.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device which is applied against a body of a subject, for implementing a primary personal care or personal health function. An imaging system is integrated into the device for imaging beneath the surface of a part of the body of the subject. The imaging system implements (the principle of) light sheet microscopy to generate an image, from which screening of disease onset or a preliminary remote diagnosis can be made, in particular relating to the vasculature beneath the surface of the part of the body.

In light sheet microscopy, the illumination beam is typically perpendicular to the imaging detection system, creating a sheet of light through a particular 2D plane of the tissue of the body part of a subject.

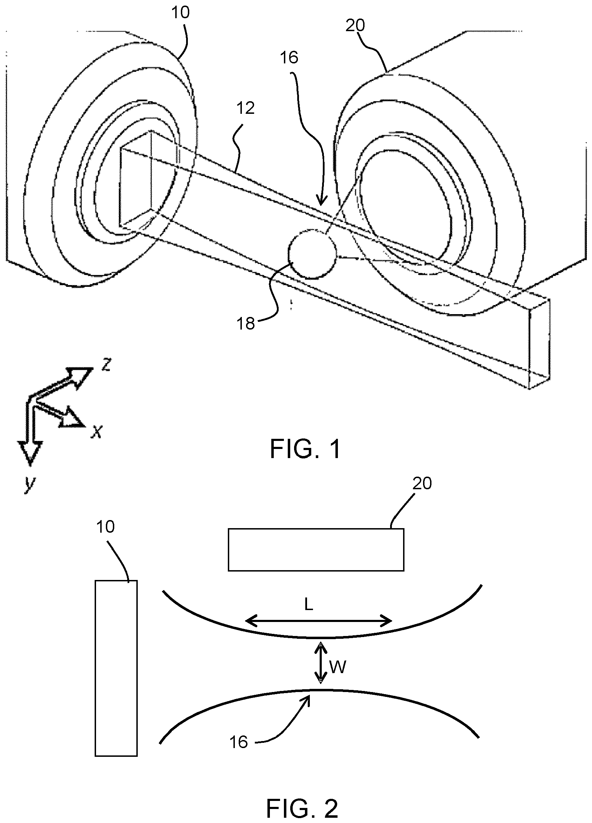

Fig. 1 shows the principle of light sheet microscopy.

An imaging system comprises an illumination source 10 for generating a light sheet 12 and directing the light sheet towards an object to be imaged, which in this disclosure is a part of the body of the subject. The illumination source for example includes a cylindrical lens or a Fresnel lens to generate the light sheet. A waist 16 of the light sheet is at a region of interest 18 of the object, and in this disclosure the region of interest 18 is beneath the surface of the part of the body.

The light wavelength is for example in the range 300 nm to 3000 nm, more preferably 600nm to 3000nm, and preferably in the infrared spectrum. The light for example penetrates by several mm's up to 1 cm beneath the surface depending on the wavelength.

A detector 20 is used to detect light from the waist of the light sheet and from a direction substantially perpendicular to a plane of the light sheet. A detected image is then generated.

This imaging configuration has multiple benefits. Firstly, a single 2D plane of the sample can be imaged quickly, meaning fast dynamic processes can be seen. Secondly, only one plane is being excited, meaning that the waist of the illumination beam lies at the position of the region of interest and there is far less out-of-focus light (when compared to widefield and confocal imaging). This improves the signal strength i.e., increases the signal to noise ratio. By imaging only a sheet in a certain depth, image data acquisition and storage volume can be reduced.

Fig. 2 shows the waist length L and width W. These are influenced by the numerical aperture, NA, and the wavelength $\lambda$ of the illumination optics, and the refractive index n of the subject's tissue:

$$W \sim \lambda / NA$$

and

$$L \sim \lambda * n / NA^2$$

Considering the case in which the illumination axis is at 90 degrees to the imaging axis, W determines the optical sectioning capability and L determines the field of view. Thus, a larger numerical aperture will give better optical sectioning capabilities at the expense of a smaller field of view.

The invention provides an imaging system as described above in which the illumination system (illumination source and objective lens) is integrated into or attached to a handheld personal care device or personal health device such as a toothbrush, any oral appliance such as mouthpiece, aligner, IPL hair removal device, epilation device, pacifier or baby bottle, breast pump, shaver, etc.

The integrated illumination source and objective lens are part of a localized high contrast light sheet imaging system, for performing light sheet contrast imaging. The detected image may then be analyzed by a health practitioner, or software may be used to analyze the image to assess deeper lying vessels such as deep veins in the leg or vessels on the under surface of the tongue or inside the breast.

A first example is for assessing deep vein thrombosis. This may be performed by an intense pulsed light, IPL, device typically used for hair removal on the legs.

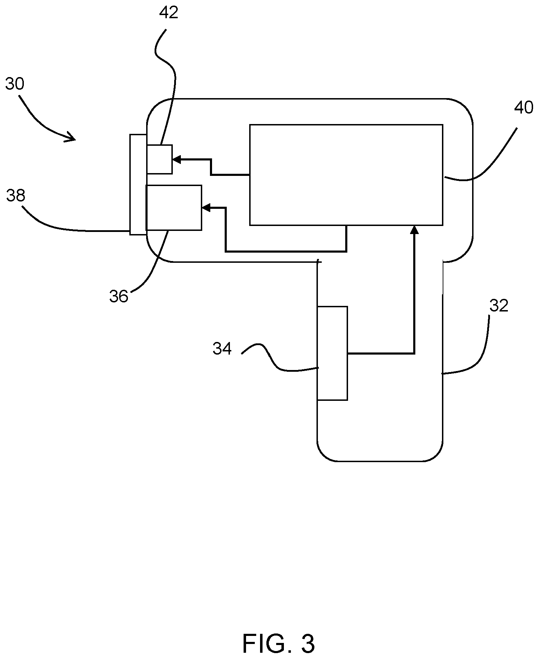

Fig. 3 shows an IPL device 30, in the form of a hand-held device having a handle 32 with a trigger 34 and a light source 36 providing IPL light through a light delivery window 38 (e.g., a sapphire window). A controller 40 controls the light source 36 to deliver light flashes to the light delivery window 38. The device also has a sensor (not shown) for detecting skin contact and/or the orientation of the device relative to the skin surface so that the light flashes are only delivered when contact is made with the skin, or images are only acquired, analyzed or stored when a certain orientation occurs.

Fig. 3 schematically shows also the imaging system as unit 42, for imaging beneath the surface of a part of the body of the subject (shown in further detail below).

For instance, during a hair removal procedure using an IPL hair removal device on the legs there is a scanning movement along the skin so that different subsurface tissue areas can be imaged over time, without the need for scanning optics. This enables real time assessment of the localization of blood clots when the vasculature is the imaging target.

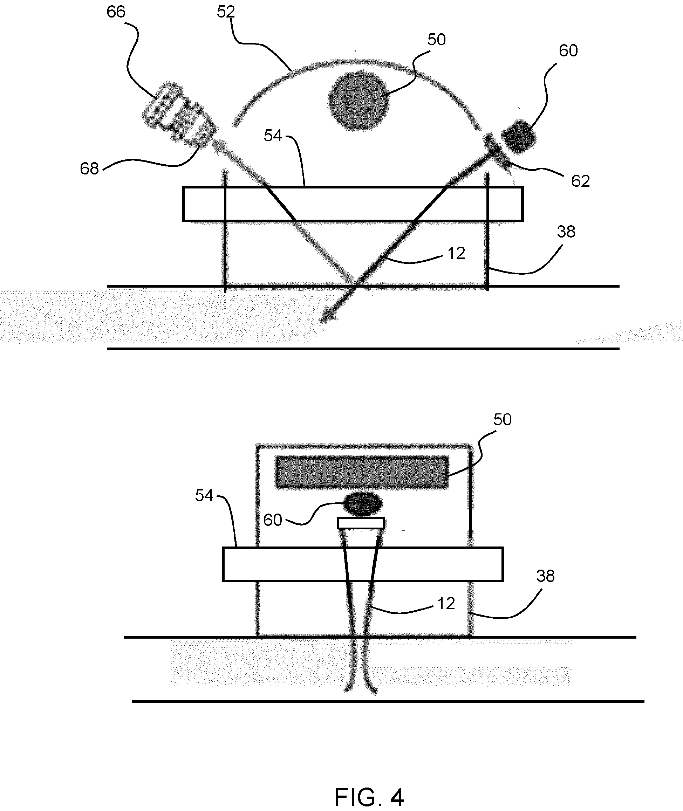

Fig. 4 shows how the imaging system 42 of Fig. 3 may be implemented. It shows the IPL light source 50 and rear reflector 52. The IPL light is delivered through a filter 54 to the light delivery window 38.

The illumination system comprises a light source 60 and illumination optics 62, which may be a cylindrical lens or a Fresnel lens, to generate the light sheet 12. The waist of the light sheet is positioned slightly outside the IPL light delivery window and hence inside the tissue. The illumination system further comprises a detector 66 having an objective lens 68.

The top image shows a side view and the bottom image shows a front view.

The IPL light delivery window 38 serves as intermediate light throughput system for the light sheet illumination and for delivery of the detection light towards the objective lens 68. The light source 60 for light sheet delivery and the objective lens 68 are close to perpendicular to each other in such a way that veins can be imaged at the working distance of the objective lens 68.

In Fig. 4, the light sheet is formed just underneath the IPL light delivery window in the skin. The light source 60 could be a LED or superluminescent laser diode. The filter 54 is a sapphire UV protector, not influencing the spectral transmittance of the LED or laser source. In this example, the light sheet passes through the filter and the detection takes place through the filter. A position shift of the illumination caused by filter should be taken into account in positioning the objective lens 68 for imaging. The object plane coincides with the light sheet that is formed inside the skin area.

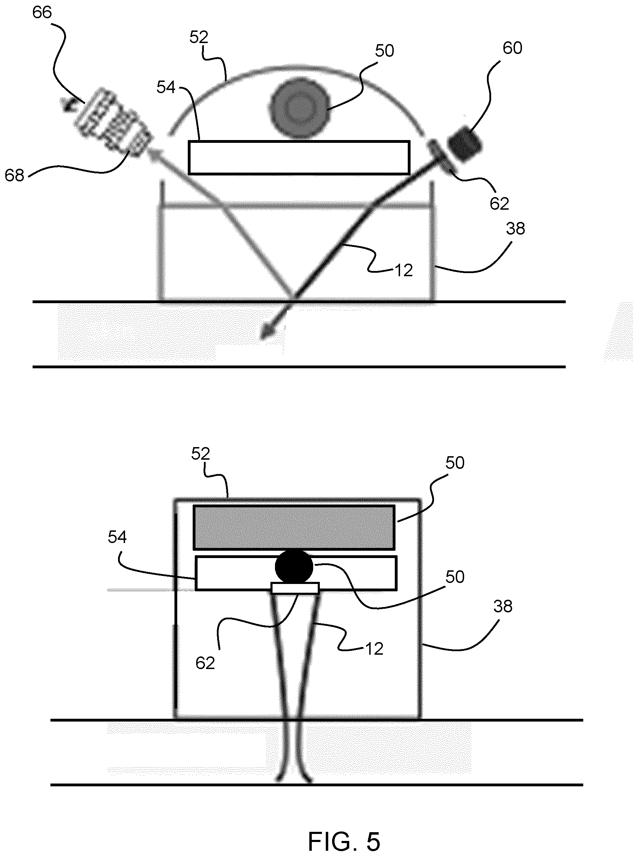

Fig. 5 shows an alternative arrangement in which the light sheet is provided laterally of the filter so only passes through the light output window. The detection also takes place without the light passing through the filter.

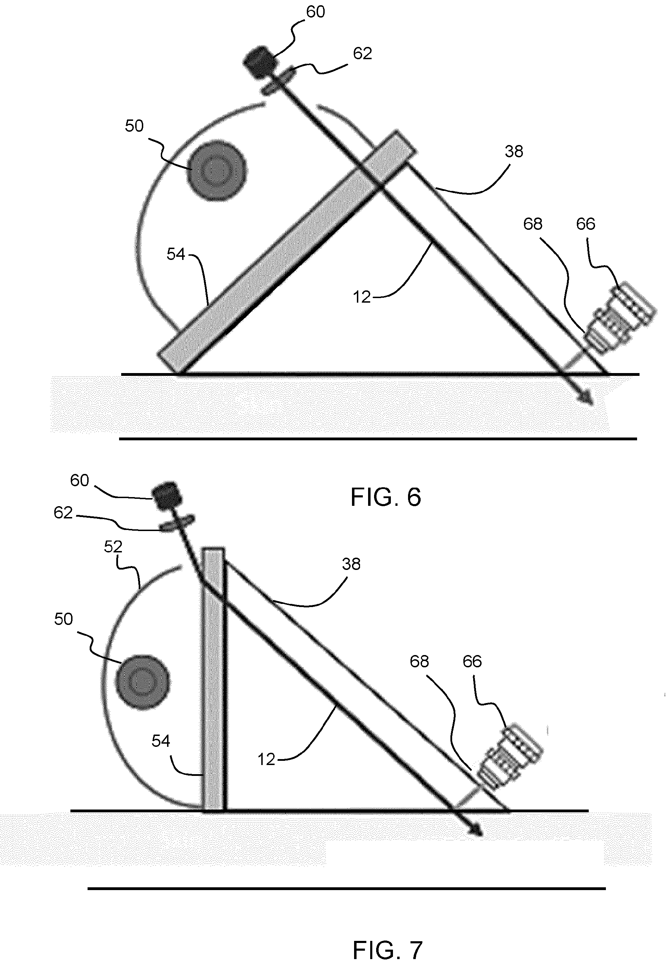

Fig. 6 shows an alternative configuration in which the light sheet 12 is delivered in a normal direction through the filter 54, and the IPL light from the light source 50 and the light sheet 12 are directed to the surface to be treated at a 45 degree angle. The detector 66 is thus also at a 45 degree angle to the surface to create the perpendicular illumination and detection paths. The light delivery window 38 has a wedge shape to define the 45 degree angle.

Fig. 7 shows a variation to Fig. 6 in which the filter 54 is perpendicular to the skin surface.

These arrangements enable the detector 66 to be closer to the skin, so that a short working distance objective lens 68 may be used. These short working distances allow for a higher detection numerical aperture, NA. The advantage of a higher NA is a higher resolution, so that smaller veins (or even arterioles, capillaries) can be imaged. Conversely, a lower NA objective lens allows for longer working distances and a wider field of view, and thus larger and/or deeper laying veins (or any other tissue structure) can be imaged.

In some examples, such as Figs. 4 and 5, the IPL illumination system and the light sheet imaging system are all in the same housing. In other examples, such as Figs. 6 and 7, the detector and its objective lens may be in a separate housing attached to the light delivery window.

A second example is for assessing sublingual varices. This may be performed by a device used in the mouth, such as a toothbrush or cleaning or whitening mouthpiece or aligner or a night guard. Sublingual varices (SLV) are dilated tortuous veins that may be seen along the ventral surface of the tongue or floor of the mouth and tend to become more prominent with age. However, in a young population, such vascular lesions could be part of Fabry, or Osler syndrome, a disorder leading to abnormal blood vessel formation in the skin.

Dilation of tongue vessels can be related to smoking, hypertension, atrial fibrillation, ischemic heart disease, myocardial infarction, stroke, other cardiovascular diseases or hemorrhagic telangiectasia and it will be an advantage of monitoring the vessels for health status.

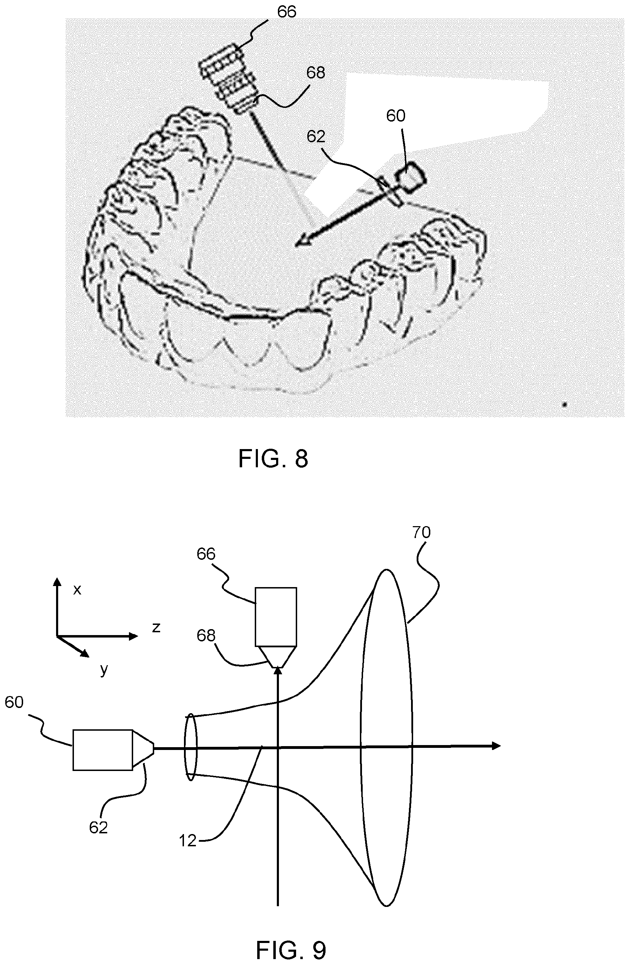

Fig. 8 shows a light sheet imaging configuration for monitoring of sublingual varices using a mouthpiece in the form of a dental aligner. It shows the light source 60 and illumination optics 62, and detector 66 and objective lens 68. The tissue surface is for example the floor of the mouth, and the illumination system is mounted on or integrated in the bottom mouthpiece, directing light downwardly. The tissue surface may be the underside of the tongue, and the illumination system may again be mounted on the bottom mouthpiece but directing light upwardly towards the tongue.

The mouthpiece for example uses a separate illumination system (e.g., using LEDs) for light-based tooth and gum treatment. Alternatively, the mouthpiece may be a clear aligner or a brushing mouthpiece. The alignment between the illumination and detection is again perpendicular in such a way that veins can be imaged by the objective lens.

The depth of the structure depends on the size of the illumination source and detection system and the distance between the two. With photonics integration, the illumination-system can be brought very close to the object of interest while still having reasonable NA, and thus reasonable resolution. Moreover, the module can be miniaturized and be made reliable and low-cost.

A third example is for assessing blood vessels such as veins of the areola or nipple capillaries, or milk ducts, in the breast. This may be performed by the shield of a breast pump or a breast massage device.

Fig. 9 shows a breast shield 70 which may be part of a breast pump. The light sheet is generated in a longitudinal direction, i.e., generally parallel to the nipple axis, and the detection direction is perpendicular. An advantage of integration into a breast pump (e.g., by overmolding integrated optical/photonics MEMS) is that the breast with the breast shield will move through a static light sheet by the pumping movement. This enables multiple images at different depths within the breast tissue to be obtained. The blood vessels and the nipple-areola complex area may be imaged to detect vasospasm or inflammation. The provision of multiple images (z-axis sectioning) is obtained by the pumping movement and not by moving parts of the imaging system.

Another aspect which may be applied to all examples above is to implement correction for the orientation of the light sheet. This may be of benefit to compensate for unevenness of the 3D tissue surface. The angle correction may use hardware and/or software. This compensation provides improved image quality and makes image quality acquisition more robust if for instance the personal care or personal health device is held against a slanted surface, in particular in order to prevent blurring or out-of-focus light. Slanted skin surfaces are commonly found all across the human body (e.g., on the leg, arms, face etc.) and are never completely even and have a certain curvature or tapering.

As a result, the target of interest (e.g., a particular depth under the skin surface) may no longer be at the focus of the objective lens. The defocus can be determined by an additional optical system (not shown).

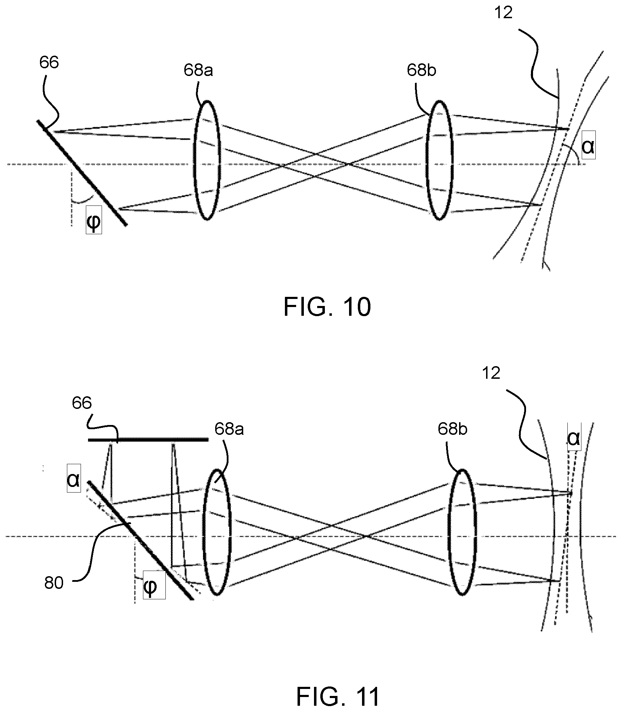

Fig. 10 shows an example where the detector 66 is tilted (by angle $\varphi$). The detector objective comprises lens 68a and 68b. These lenses are designed to focus a slanted light sheet 12 (slanted with angle $\alpha$) so that all points along the waist of the light sheet are in focus.

Thus, the issue of an out-of-focus image resulting from a tilted surface (differing or deviating from the angle $\alpha$) can be compensated by tilting the sensor. A fixed tilted surface can only provide a focused image for a particular slant of the surface being imaged, and normally the hand-held device moves over a contoured skin surface area where tilt will change over the area.

Fig. 11 shows a solution with a compensating scanning mirror 80 to achieve the proper focus on the sensor 66 with compensation of the tilt at the skin area.

An additional optical system for measuring the focus of the captured image may be used to provide a feedback correction signal for driving the mirror 80. This focus measurement system is not shown, but comprises conventional image processing. Alternatively, orientation sensing may be used to detect the slant.

For a small-tilted angle $\alpha$, the orientation of the illumination unit does not need to be adapted and the light sheet will capture the focal plane. For large tilt angles the imaging system needs to be adjusted. Many hand-held devices have an inertial measurement unit (IMU), accelerometer, gyroscope or in the future maybe also an optical guidance dot producing light patterns that can be linked to a certain device orientation relative to the to-be-imaged tissue surface.

Three concepts based on the use of IMU information relating to the spatial orientation of the personal health device relative to the skin surface will be described below.

A first concept is to adapt or adjust the orientation of the detector and/or the illumination optics. The orientation information may be obtained by an electronically configurable 3-axis or 6-axis tilting stage (or even a gimbal). In one example, the control loop comprises a controller adjusting in real-time the orientation of the detector objective based on the measured orientation of the personal health or personal care device.

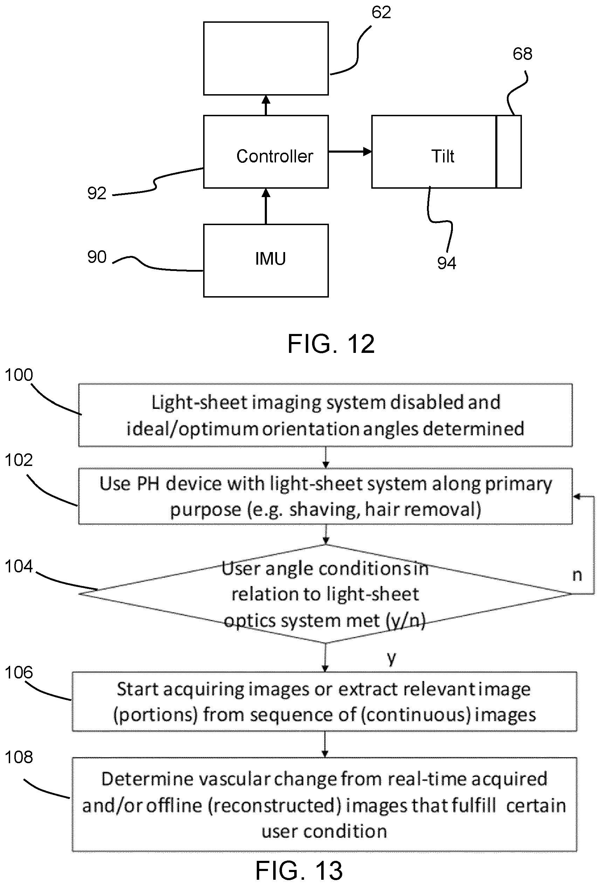

Fig. 12 shows an IMU 90 providing motion sensing information to a controller 92 which then controls a tilt stage 94, such as a piezoelectric tilt stage, to which the detection objective 68 is mounted. Furthermore, the controller controls settings of the illumination system 62 (in response to the measured orientation or just based on predetermined characteristics).

A second concept, shown in Fig. 13, is to store and/or analyze images only if the hand-held device with light-sheet imaging system has the theoretically ideal orientation relative to the tissue surface.

In step 100, the light sheet imagine system is disabled and the ideal orientation angles are determined.

In step 102, the device is used for its primary purpose, such as shaving or other hair removal.

In step 104, it is tested if the angle conditions are met while the normal use takes place. If the angle conditions are not met, the method angle conditions are tested repeatedly while the normal use takes place.

Wheen the angle conditions are met, images are acquired in step 106, or relevant images or image portions are extracted from a sequence of continuous images.

Vascular changes can then be determined (or other conditions being investigated) in step 108 in real time or offline.

In this way, an algorithm is used to extract or acquire only images that fulfil certain conditions of angles measured with an IMU or single guidance dot concept (using a prior definition of ideal combinations of IMU angles or guidance dot patterns). Angle conditions are tested during use of the device, and if those angle conditions are met, images are acquired, or relevant image portions are extracted from a sequence of continuous images.

A third concept is to reconstruct sharp images from an image sequence and stitch together in-focus sections of each image to build up a perfectly sharp image.

The advantage of these approaches is that they enable higher image quality pictures (in that only pixels in focus are analyzed or imaged) at reduced computational power (with reduced data storage rate, and reduced data volume). For instance, the image will only be stored and analyzed for situations when the detector is at 90° relative to tissue surface.

In all cases, vascular changes may be determined from real-time acquired images and/or offline reconstructed images that fulfill certain user conditions.

The invention is not limited to analysis of the vasculature. Light sheet imaging can be used for any anatomical structure if the resolution is sufficient, by using a suitable selection of wavelength, i.e., taking blood absorption into account, and penetration depth. For imaging milk vessels, fat absorption is taken into account. The imaging could be of lymph nodes, glands etc.

Light sheet fluorescence imaging may for example be used for imaging fungus grown in tissue.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A handheld personal care or personal health device, comprising:

   a user interfacing element for application against a body of a subject, for implementing a primary personal care or personal health function; and
   an imaging system for imaging beneath the surface of a part of the body of the subject, wherein the imaging system comprises:

   an illumination source (60,62) for generating a light sheet and directing the light sheet towards the part of the body such that a waist of the light sheet is at a region of interest beneath the surface of the part of the body; and
   a detector (66,68) for detecting light from the waist of the light sheet from a direction substantially perpendicular to a plane of the light sheet and generating a detected image.

2. The device of claim 1, wherein the illumination source comprises a light source and illumination optics for generating the light sheet and the detector comprises detection optics focused at the waist of the light sheet and a detector.

3. The device of claim 2, wherein the detection optics comprises an objective lens.

4. The device of claim 2 or 3, wherein one or both of the illumination optics and the detection optics comprise optical filters.

5. The device of any one of claims 2 to 4, comprising:

   a sensor for sensing an incline of the part of the body of the subject; and
   a controller for adjusting illumination optics and/or the detection optics in dependence on the sensed incline.

6. The device of claim 1 to 4, comprising:

a sensor for sensing an incline of the part of the body of the subject; and

a controller for controlling the storage and/or analysis of the detected images in dependence on the sensed incline or for combining image portions to create an overall image based on the sensed incline of the image portions.

7. The device of claim 1 to 4, comprising:

a sensor for sensing an incline of the part of the body of the subject;

a scanning mirror between the detection optics and the detector; and

a controller for controlling the scanning mirror orientation in dependence on an incline of the part of the body of the subject.

8. The device of any one of claims 1 to 7, wherein the imaging system is for imaging the vasculature beneath the surface of the subject.

9. The device of claim 8, further comprising a processor for analyzing the detected image to provide an assessment of vascular diseases or abnormalities such as deep vein thrombosis, varicose veins, spider veins, vasospasm or inflammation etc.

10. The device of claim 9, comprising an intense pulsed light unit of an epilator, and the primary personal care or personal health function comprises hair removal such as epilation.

11. The device of claim 10, wherein the intense pulsed light unit of the epilator has a light delivery window for delivering light flashes, wherein the illumination source delivers the light sheet through the light delivery window.

12. The device of claim 8, further comprising a processor for analyzing the detected image to provide an assessment of sublingual varices.

13. The device of claim 12, comprising an oral appliance such as mouthpiece, and the primary personal care or personal health function comprises gum and/or tooth treatment.

14. The device of claim 8, further comprising a processor for analyzing the detected image to provide an assessment breast vasospasm or inflammation.

15. The device of claim 14, comprising a breast pump, and the primary personal care or personal health function comprises breast pump milk extraction.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

Europäisches
Patentamt
European
Patent Office
Office européen
des brevets

# EUROPEAN SEARCH REPORT

**Application Number**

**EP 23 21 1258**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/154382 A1 (ALTSHULER GREGORY B [US] ET AL) 14 July 2005 (2005-07-14) * paragraphs [0002] - [0021] * * paragraph [0043] * * paragraphs [0090] - [0102] * * paragraphs [0126] - [0127] * | 1-4, 10-15 | INV. A61B5/00 A45D26/00 A61B18/20 A61C17/16 A61M1/06 |
| A | US 2017/027454 A1 (KORPOSH SERGIY [GB] ET AL) 2 February 2017 (2017-02-02) * paragraphs [0002] - [0006] * * paragraph [0065] * | 1-15 | |
| A | WO 2014/207003 A1 (KONINKL PHILIPS NV [NL]) 31 December 2014 (2014-12-31) * the whole document * | 1-15 | |
| A | US 2023/149077 A1 (VAN GOOL EDGAR [NL] ET AL) 18 May 2023 (2023-05-18) * paragraphs [0001] - [0015] * * paragraph [0045] * * paragraphs [0067] - [0074] * | 1-15 | TECHNICAL FIELDS SEARCHED (IPC) |
| A | US 2008/060148 A1 (PINYAYEV ALEKSEY M [US] ET AL) 13 March 2008 (2008-03-13) * paragraphs [0003] - [0005] * * paragraph [0061] * * paragraph [0142] * * paragraph [0268] * | 12,13 | A61B A61M A61C |

The present search report has been drawn up for all claims

| Place of search | Date of completion of the search | Examiner |
|---|---|---|
| The Hague | 26 April 2024 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 1258

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2024

| Patent document cited in search report | Publication date | Patent family member(s) | Publication date |
|---|---|---|---|
| US 2005154382 A1 | 14-07-2005 | US 2005154381 A1 | 14-07-2005 |
| | | US 2005154382 A1 | 14-07-2005 |
| | | US 2008147054 A1 | 19-06-2008 |
| | | WO 2005065565 A1 | 21-07-2005 |
| US 2017027454 A1 | 02-02-2017 | EP 3128900 A1 | 15-02-2017 |
| | | US 2017027454 A1 | 02-02-2017 |
| | | WO 2015155526 A1 | 15-10-2015 |
| WO 2014207003 A1 | 31-12-2014 | CN 105338888 A | 17-02-2016 |
| | | CN 112914515 A | 08-06-2021 |
| | | EP 3013213 A1 | 04-05-2016 |
| | | JP 6509829 B2 | 08-05-2019 |
| | | JP 2016526408 A | 05-09-2016 |
| | | US 2016120604 A1 | 05-05-2016 |
| | | WO 2014207003 A1 | 31-12-2014 |
| US 2023149077 A1 | 18-05-2023 | BR 112022021172 A2 | 06-12-2022 |
| | | CN 115426919 A | 02-12-2022 |
| | | EP 3900574 A1 | 27-10-2021 |
| | | EP 4138611 A1 | 01-03-2023 |
| | | ES 2959084 T3 | 20-02-2024 |
| | | IL 297360 A | 01-12-2022 |
| | | JP 2023522235 A | 29-05-2023 |
| | | KR 20230002867 A | 05-01-2023 |
| | | PL 4138611 T3 | 05-02-2024 |
| | | US 2023149077 A1 | 18-05-2023 |
| | | WO 2021213852 A1 | 28-10-2021 |
| US 2008060148 A1 | 13-03-2008 | AU 2005329104 A1 | 21-09-2006 |
| | | CA 2600855 A1 | 21-09-2006 |
| | | CN 101193605 A | 04-06-2008 |
| | | EP 1855612 A1 | 21-11-2007 |
| | | JP 2008532619 A | 21-08-2008 |
| | | US 2008060148 A1 | 13-03-2008 |
| | | WO 2006098719 A1 | 21-09-2006 |

EPO FORM P0459

For more details about this annex : see Official Journal of the European Patent Office, No. 12/82